# EUROPÄISCHE PATENTANMELDUNG

(11) **EP 2 685 396 A1**
(43) Veröffentlichungstag der Anmeldung: **15.01.2014**
(21) Anmeldenummer: 12176452.6
(22) Anmeldetag: 13.07.2012
(51) Int. Cl.: G06F 19/00, G06Q 50/22

(54) **Selbstkontrollierendes Dokumentationssystem für validiertes Verfahren**

(71) Anmelder: IMC Systems GmbH, 55118 Mainz (DE)
(72) Erfinder: Buß, Wolfgang, 55118 Mainz (DE)
(74) Vertreter: Bittner, Peter

(57) **Zusammenfassung**

Computersystem, computerimplementiertes Verfahren und Computerprogramprodukt (1000) zur Überwachung der Aufbereitung von Medizinprodukten nach einem validierten Verfahren. Ein Aufzeichnungsmodul (1001) ist konfiguriert Messwerte (M1, M2) von mindestens einem im validierten Verfahren verwendeten Aufbereitungsmittel (2001, 2002) zu empfangen, wobei die Messwerte für das validierte Verfahren relevante technische Parameter (101, 102) umfassen. Ein Dokumentationsmodul (1002) ist konfiguriert zum Erfassen mindestens einer Freigabeentscheidung in Bezug auf mindestens eine Arbeitsanweisung hinsichtlich der verwendeten Aufbereitungsmittel (2001, 2002) des validierten Verfahrens. Das Dokumentationsmodul (1002) verhindert zumindest teilweise eine Annahme der Freigabeinstruktion, wenn zumindest ein Messwert (M1, M2) eines für das validierte Verfahren relevanten technischen Parameters (101, 102) außerhalb eines vordefinierten Toleranzbereichs liegt.

## Beschreibung

### Technisches Gebiet

Die Erfindung bezieht sich auf elektronische Datenverarbeitung im Allgemeinen und im Speziellen auf ein Computersystem, Computer-Programmprodukt und computerimplementiertes Verfahren zur Überwachung validierter Aufbereitungsverfahren, im Folgenden auch validierte Verfahren genannt.

### Stand der Technik

In Gesundheitseinrichtungen kommt es immer wieder zu Infektionen bei Patienten, die auf den Einsatz verunreinigter Medizinprodukte bei deren Behandlung zurückzuführen sind. Deshalb ist es notwendig, dass solche Medizinprodukte nach einem validierten Aufbereitungsverfahren aufbereitet werden, bevor sie beim Patienten zum Einsatz kommen.

Geeignete validierte Verfahren sind Verfahren, welche ein definiertes Ergebnis (insbesondere Sauberkeit, Keimarmut/Sterilität und Funktionalität) reproduzierbar und nachweisbar ständig erbringen. Bei der Aufbereitung eines Medizinprodukts trägt die Summe aller beteiligten maschinellen und manuellen Prozesse (Einzelschritte der Aufbereitung) zum Erreichen des jeweiligen Aufbereitungsziels bei. Insoweit wirken sich unzulänglich validierte Einzelschritte (Prozesse) ebenso qualitätsmindernd auf das Ergebnis der Aufbereitung aus, wie die Nichtbeachtung von Standardarbeitsanweisungen.

Um die Qualität der Prozesse und ein dauerhaft gleichbleibendes Verfahren sicher zu stellen, sind folgende Schritte hinsichtlich eines aufzubereitenden Medizinprodukts gemäß der Empfehlung für die Überwachung der Aufbereitung von Medizinprodukten (erstellt von der Projektgruppe "RKI-BfArM-Empfehlung" der Arbeitsgruppe Medizinprodukte AGMP) zu beachten (dabei ist generell für jeden Schritt eine Standardarbeitsanweisung zu erstellen und eine Dokumentation zu erfassen, dass der Schritt erfolgreich gemäß Arbeitsanweisung ausgeführt wurde).
- Vorbehandeln
- Sammeln
- Vorreinigen
- Zerlegen
- Reinigung, Desinfektion: Bei manueller R/D (Reinigung/Desinfektion): Standardarbeitsanweisung erstellen; bei maschineller R/D: Prozessvalidierung durchführen
- Spülung, Trocknung
- Prüfung auf Sauberkeit/Unversehrtheit
- Pflege, Instandsetzung
- Funktionsprüfung: primär Standardarbeitsanweisung, in speziellen Fällen Prozessvalidierung erforderlich
- Kennzeichnung
- Verpackung: Standardarbeitsanweisung, bei Einschweißen Prozessvalidierung durchführen
- Sterilisation: Prozessvalidierung durchführen Dokumentierte Freigabe
- Standardarbeitsanweisung zur Schnittstellenregelung (z. B. Vorgaben zur Reinigung und Desinfektion, Übergabe, Transport, Lagerung)
- Standardarbeitsanweisung zum Umgang mit Abweichungen/Fehlern

Bestehende computergestützte Lösungen zur Durchführung dieses validierten Verfahrens sind in der Lage, technische Parameter der im Verfahren eingesetzten Verfahrensschritte zu sammeln und zu dokumentieren und von einem qualifizierten Benutzer eine Freigabeentscheidung zu erfassen.

Dabei ist dem Benutzer vollständig die technische Aufgabe angelastet, die ordnungsgemäße Durchführung des Aufbereitungsverfahrens für das jeweilige Medizinprodukt als Grundlage für seine Freigabeentscheidung zu überprüfen.

Insbesondere beim Einsatz komplexer Aufbereitungsmittel, wie zum Beispiel computergesteuerte Sterilisatoren oder Desinfektionsgeräte in Kombination mit manuellen Teilschritten, ist es für einen Benutzer nicht möglich, eine vollständige Überprüfung hinsichtlich aller für das validierte Aufbereitungsverfahren relevanten Parameter durchzuführen, da die Benutzer eines Dokumentationssystems für das validierte Aufbereitungsverfahren in der Regel medizinische Assistenzkräfte sind, deren technische Vorbildung eine technische Bewertung komplexer technischer Abläufe nicht zulässt.

### Überblick

Es besteht daher ein Bedarf für ein computergestütztes System zur Überwachung der Aufbereitung von Medizinprodukten nach einem validierten Verfahren, welches einen Benutzer bei der Überprüfung der ordnungsgemäßen Ausführung technischer, sowohl automatisierter als auch manueller Teilschritte des validierten Verfahrens dahingehend unterstützt, dass der Benutzer unsachgemäß bearbeitete Medizinprodukte nicht zur erneuten Verwendung bei der Patientenbehandlung freigibt.

Dieses technische Problem wird durch die technischen Merkmale der unabhängigen Ansprüche in Form eines Computersystems, eines Computerprogrammprodukts und eines computer-implementierten Verfahrens erfindungsgemäß gelöst.

Das erfindungsgemäße Computersystem zur Überwachung der Aufbereitung von Medizinprodukten nach einem validierten Verfahren umfasst dabei ein Aufzeichnungsmodul, welches konfiguriert ist Messwerte von mindestens einem im validierten Verfahren verwendeten Aufbereitungsmittel zu empfangen. Die Messwerte umfassen für das validierte Verfahren relevante technische Parameter, wie beispielsweise Druck oder Temperatur, welche während eines Aufbereitungsschritts in dem jeweiligen Aufbereitungsmittel herrschten. Weiterhin umfasst das Computersystem ein Dokumentationsmodul zum Erfassen mindestens einer Freigabeinstruktion für eine Freigabeentscheidung in Bezug auf mindestens eine Arbeitsanweisung hinsichtlich der verwendeten Aufbereitungsmittel des validierten Verfahrens. Die Freigabeentscheidung durch das Computersystem basiert dabei auf technischen Überlegungen zur Laufzeit, die einer technischen Qualitätskontrolle des freizugebenden Aufbereitungsschritts entsprechen. Dies umfasst, dass ein Benutzer, der für die Freigabeentscheidung verantwortlich ist, den ordnungsgemäßen technischen Ablauf des freizugebenden Schritts zweifelsfrei überprüfen und validieren muss und dem Computersystem schließlich eine Freigabeinstruktion erteilt. Die Freigabeentscheidung erfordert eine Überprüfung und Dokumentation des vollständigen und korrekten Prozessverlaufs des validierten Verfahrens. Das Computersystem umfasst weiterhin ein Auswertungsmodul, welches erfindungsgemäß so konfiguriert ist, dass es eine Annahme der Freigabeinstruktion zumindest teilweise verhindert, wenn zumindest ein Messwert eines für das validierte Verfahren relevanten technischen Parameters außerhalb eines vordefinierten Toleranzbereichs liegt. Eine teilweise Verhinderung der Annahme der vom Nutzer erteilten Freigabeinstruktion bedeutet, dass ggf. eine Freigabe für bestimmte Teilschritte des validierten Verfahrens erfolgt, das aber keine vollständige Freigabe erteilt werden kann. bei einer solchen Verhinderung ist daher immer noch eine Nachfolgeaktivität erforderlich, bei der die nicht freigegebenen Prozessschritte noch eine weitere Bearbeitung erfahren.

Die Messwerte können dabei über entsprechende Maschinenschnittstellen direkt vom Aufbereitungsmittel an das Computersystem übertragen werden. In manchen Fällen ist auch eine manuelle Eingabe von Messwerten durch den Benutzer möglich.

Der Benutzer wird somit daran gehindert, eine Freigabeentscheidung für Verfahrensschritte des validierten Verfahrens zu erteilen, bei denen nicht der technisch erforderliche Ablauf des Aufbereitungsverfahrens gewährleistet war, selbst wenn der Benutzer eine entsprechende Freigabeinstruktion erteilt. Die Überprüfung der für die Freigabe eines validierten Verfahrensschritts erforderlichen technischen Voraussetzungen erfolgt dabei durch das Computersystem in Echtzeit mit einer im Vergleich zu einer vom Benutzer durchgeführten Überprüfung deutlich verringerten Fehlerwahrscheinlichkeit. Die erfindungsgemäß aufbereiteten Medizinprodukte sind daher mit einer an Sicherheit grenzenden Wahrscheinlichkeit für eine erneute Verwendung entsprechend aufbereitet und können außerdem der Verwendung im Behandlungsprozess schneller wieder zugeführt werden, da eine langwierige und fehleranfällige manuelle Überprüfung der technischen Parameter durch den Benutzer entfällt. Der damit beschleunigte Aufbereitungszyklus führt auch dazu, dass z.B. eine Arztpraxis weniger Medizinprodukte eines bestimmten Typs benötigt, da durch die schnellere Wiederverfügbarkeit weniger zwischengelagerte aufbereitete Medizinprodukte dieses Typs erforderlich sind. Weiterhin erhält der Benutzer in Form entsprechender Benutzerführung durch das System Unterstützung, die ihm dabei hilft, seine technische Aufgabe der Freigabe mit einer verbesserten Genauigkeit durchzuführen.

Optional umfasst das Computersystem weiterhin ein Bibliotheksmodul, welches für die verwendeten Aufbereitungsmittel wählbare Programme vorhält, wobei ein wählbares Programm einen zu erwartenden zeitlichen Messverlauf für mindestens einen der relevanten technischen Parameter enthält, sowie ein Auswertungsmodul, welches konfiguriert ist, einen Soll-lst-Vergleich für mindestens einen zu erwartenden technischen Parameter eines gewählten Programms mit mindestens einem entsprechenden empfangenen Messwert auf der Basis des vordefinierten Toleranzbereichs durchzuführen.

Somit kann das Computersystem eine Vielzahl an Aufbereitungsmitteln unterstützen, wobei jedes dieser Aufbereitungsmittel in verschiedenen Betriebsmodi eingesetzt werden kann. Unterstützt ein Aufbereitungsmittel (z.B. Sterilisator) beispielsweise die Sterilisation von Medizinprodukten unterschiedlicher Risikoklassen, so kann für jede dieser Risikoklassen ein spezielles Betriebsprogramm erforderlich sein. Die verschiedenen Betriebsprogramme für sämtliche verwendeten Aufbereitungsmittel können nun im Besagten Bibliotheksmodul frei wählbar gespeichert sein. Jedes der frei wählbaren Programme enthält dabei zumindest den zu erwartenden Messverlauf der für den ordnungsgemäßen Ablauf des validierten Verfahrens relevanten technischen Parameter des jeweiligen Aufbereitungsmittels für das jeweilige Medizinprodukt oder für die entsprechende Risikoklasse. Das Auswertungsmodul ermöglicht dann einen Soll-Ist-Vergleich der Messwerte mit dem erlaubten Toleranzbereich. Findet dieser Soll-Ist-Vergleich in Echtzeit statt, so kann zu jedem Zeitpunkt ein Schritt des validierten Verfahrens bei einer Fehlfunktion vom Computersystem unmittelbar abgebrochen werden, was wiederum eine Beschleunigung des Aufbereitungsverfahrens zur Folge hat und den Benutzer von der Überprüfung eines umsonst durchgeführten Aufbereitungsschrittes entlastet.

In einer Ausführungsform kann das Dokumentationsmodul eine Warnung für den Benutzer generieren, wenn zumindest ein Messwert eines für das validierte Verfahren relevanten technischen Parameters in einen kritischen Bereich des vordefinierten Toleranzbereichs fällt. Eine solche Warnung ermöglicht beispielsweise dem Benutzer ein frühzeitiges, korrigierendes Eingreifen in einen laufenden Aufbereitungsschritt. Der technische Parameter liegt in diesem Fall noch innerhalb des vordefinierten Toleranzbereichs, jedoch kann eine gewisse Gefahr bestehen, dass der Aufbereitungsprozess nicht erwartungsgemäß läuft und ohne eine Korrektivmaßnahme der entsprechende Schritt nicht zu einem sachgemäßen Erfolg führt. Durch die generierte Warnung kann der Benutzer eingreifen und sicherstellen, dass das Verfahren zum gewünschten Erfolg führt, was wiederum einen schnelleren Ablauf des Gesamtverfahrens und der letztendlich erforderlichen Freigabe bewirkt, da eine Wiederholung des ansonsten nicht freigabefähigen Schrittes vermieden werden kann.

Weitere Aspekte der vorliegenden Erfindung sind ein computer-implementiertes Verfahren, welches durch das beschriebene Computersystem ausgeführt werden kann, sowie ein Computerprogrammprodukt mit Instruktionen, die vom besagten Computersystem derart abgearbeitet werden können, so dass das validierte Verfahren entsprechend ausgeführt wird.

Weitere vorteilhafte Ausführungsformen der Erfindung finden sich in den abhängigen Ansprüchen wieder.

### Kurzbeschreibung der Zeichnungen

FIG. 1 zeigt ein Ausführungsbeispiel des erfindungsgemäßen Computersystems mit Peripheriegeräten;
FIG. 2 ist ein Flussdiagramm zur Veranschaulichung eines erfindungsgemäßen Verfahrens;
FIG. 3. zeigt eine Darstellung von aufgezeichneten von einem Aufbereitungsmittel empfangenen Messwerten relevanter technischer Verfahrensparameter;
FIG. 4 ist eine schematische Darstellung eines grafischen Benutzerinterfaces des erfindungsgemäßen Computersystems; und
FIG. 5 zeigt vereinfachte Datenstrukturen, die im erfindungsgemäßen Computersystem gekoppelt sind.

### Detailbeschreibung

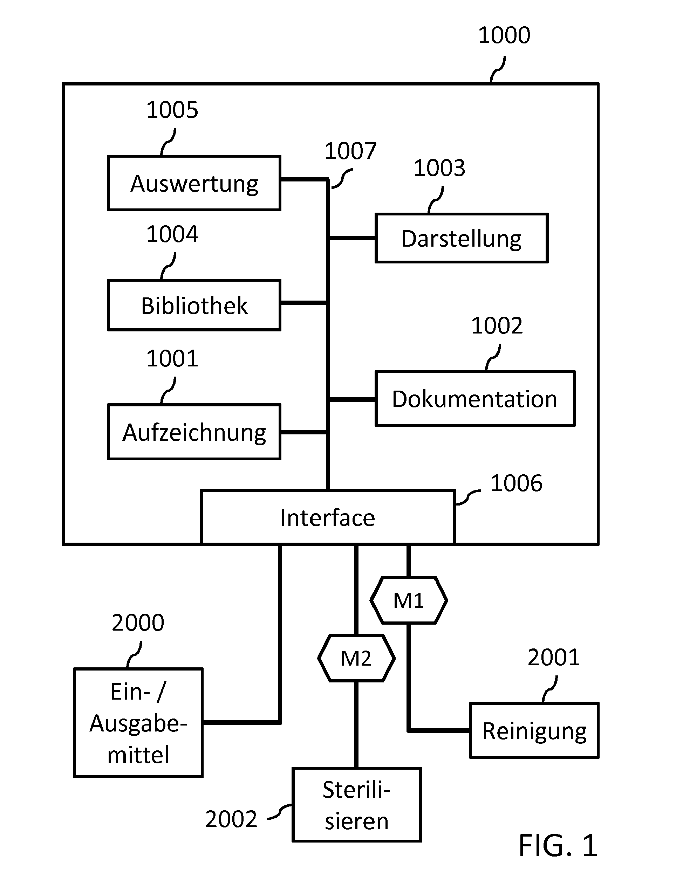

FIG. 1 zeigt ein Ausführungsbeispiel des erfindungsgemäßen Computersystems 1000 mit Peripheriegeräten 2000, 2001, 2002. Das Computersystem 1000 dient zur Überwachung der Aufbereitung von Medizinprodukten nach einem validierten Verfahren. Eine ordnungsgemäße Aufbereitung erfordert dabei die Beachtung der gemeinsamen Empfehlung der Kommission für Krankenhaushygiene und Infektionsprävention beim Robert Koch-Institut und des Bundesinstitutes für Arzneimittel und Medizinprodukte zu den Anforderungen an die Hygiene bei der Aufbereitung von Medizinprodukten. Medizinprodukte sind Instrumente oder Apparate, die zur Anwendung an den Menschen zum Zwecke der Erkennung, Überwachung, Verhütung, Behandlung oder Linderung von Krankheiten bestimmt sind. Weiter Details zu gesetzlichen Vorschriften zur Infektionsprävention finden sich in der Medizinprodukte-Betreiberverordnung in der Fassung der Bekanntmachung vom 21. August 2002 (BGBI. I S. 3396), die zuletzt durch Artikel 4 des Gesetzes vom 29. Juli 2009 (BGBI. I S. 2326) geändert worden ist. Die Aufbereitung von keimarm oder steril zur Anwendung kommenden Medizinprodukten wird mit einem geeigneten validierten Verfahren so durchgeführt, dass der Erfolg dieser Verfahren nachvollziehbar gewährleistet ist. Aufbereitung beinhaltet dabei sachgerechte Vorbereitung (u.a. zügiger Transport, Schutz vor Beschädigung), Reinigung/Desinfektion, Spülung und Trocknung, Prüfung auf Sauberkeit und Unversehrtheit, Pflege und Instandsetzung, Funktionsprüfung, Verpacken und Kennzeichnung und Sterilisation. Beispielsweise müssen verwendete Desinfektionsverfahren nachweislich bakterizid, fungizid und viruzid sein und sicherstellen, dass keine Fixierung von Rückständen oder Proteinen eintritt. Spülung und Trocknung müssen Reinigungs- und Desinfektionsmittellösung durch intensives Nachspülen durch Wasser mit mindestens Trinkwasserqualität entfernen so dass eine Rekontamination ausgeschlossen wird.

Die Medizinprodukte sind dabei in der Regel in Klassen eingeteilt, die einen Indikator für das Kontaminationsrisiko des jeweiligen Medizinprodukts darstellen. Zur Klasse der unkritischen Medizinprodukte gehören solche, die lediglich mit intakter Haut in Berührung kommen, wie z.B. Blutdruckmessgeräte oder EKG-Elektroden. Zur Klasse der semikritischen Medizinprodukte, gehören solche. die mit Schleimhaut oder krankhaft veränderter Haut in Berührung kommen. Dabei gibt es eine weitere Untergliederung in eine Gruppe A ohne besondere Anforderungen an die Aufbereitung (z.B. Spekulum) und in eine Gruppe B mit erhöhten Anforderungen an die Aufbereitung (z.B. Endoskop). Zur Klasse der kritischen Medizinprodukte gehören Produkte zur Anwendung von Blut, Blutproben, sterilen Arzneimitteln sowie Produkte, die Haut oder Schleimhaut durchdringen und Kontakt mit Blut, inneren Geweben oder Organen, einschließlich Wunden haben. Bei dieser Klasse gibt es zusätzlich eine Gruppe mit besonders hohen Anforderungen an die Aufbereitung. Das Computersystem kann eine Zuordnungsstruktur speichern, in der für jedes Medizinprodukt, welches mit dem validierten Verfahren aufbereitet werden kann, die Zuordnung zur jeweiligen Risikoklasse gespeichert ist.

Ein beispielhafter Aufbereitungsprozess umfasst Vorbereitung, Reinigung, Desinfektion incl. Spülen und Trocknen, Kontrolle aller Schritte, Siegelgerät (Verpackung), Sterilisation, Kennzeichnung und Freigabe. Der Aufbereitungsprozess ist validiert, wenn eine Standardarbeitsanweisung für jeden Schritt der Aufbereitung existiert, der Prozess reproduzierbar ist, objektivierte Parameter den jeweiligen, gewünschten Erfolg belegen und jeder Schritt der Aufbereitung dokumentiert ist.

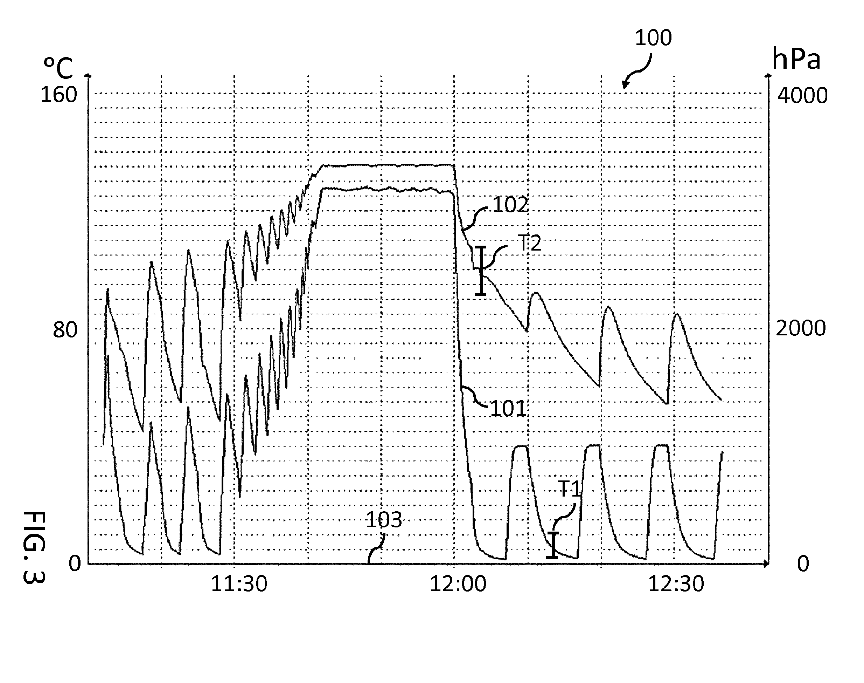

Das Computersystem 1000 umfasst ein Aufzeichnungsmodul 1001, welches konfiguriert ist Messwerte M1, M2 von mindestens einem im validierten Verfahren verwendeten Aufbereitungsmittel 2001, 2002 zu empfangen, wobei die Messwerte für das validierte Verfahren relevante technische Parameter umfassen. Beispiele für Aufbereitungsmittel sind Vorrichtungen wie Reinigungsgeräte 2001, Desinfektionsapparate, Trocknungsgeräte, Kontroll-, Wartungs- und Prüfungsgeräte, Verpackungsmaschinen oder Sterilisationsapparate 2002. Beispiele für das validierte Verfahren relevante technische Parameter sind Druck, Temperatur, Zeitdauer und Prüfindikatorzustand. Die Messwerte M1, M2 können vom Computersystem über ein entsprechendes Interface 1006 von den Aufbereitungsmitteln empfangen werden und dem Aufzeichnungsmodul 1001 zur Verfügung gestellt werden. Zum Beispiel kann das Aufzeichnungsmodul über das Interface 1006 die Daten via eine serielle oder parallele Standard-Maschinenschnittstelle (z.B. RS 232, USB, FireWire, LPT, etc.)vom verwendeten Aufbereitungsmittel 2001, 2002 empfangen. Das Aufzeichnungsmodul ist dabei kommunikativ mit dem Interface 1006 gekoppelt. Dies kann z.B. mittels entsprechender Modulschnittstellen 1007 realisiert werden. Das Aufzeichnungsmittel kann die empfangenen Messwerte in Echtzeit aufzeichnen. Dies kann beispielsweise in tabellenartigen Strukturen oder in XML (extensible markup language) Dateien erfolgen. Andere dem Fachmann bekannte Datenablagemöglichkeiten können dafür ebenso Verwendung finden. Die Datenstrukturen mit den aufgezeichneten Daten können dabei in jedem dafür geeigneten Speichermedium abgelegt werden, wobei es nicht darauf ankommt, ob es sich um einen Persistenzspeicher (z.B. SSD-Speichermodul, Festplatte) zur dauerhaften Ablage oder um ein flüchtiges Speichermedium (z.B. RAM) handelt. FIG. 3 zeigt Beispiele von Messdaten 101, 102, die vom Aufzeichnungsmodul empfangen wurden.

Weiterhin umfasst das Computersystem ein Dokumentationsmodul 1002 zum Erfassen mindestens einer Freigabeentscheidung in Bezug auf mindestens eine Arbeitsanweisung hinsichtlich der verwendeten Aufbereitungsmittel 2001, 2002 des validierten Verfahrens. Dabei kann das Dokumentationsmodul Arbeitsanweisungen für jeden im validierten Verfahren erforderlichen Schritt beinhalten. Die entsprechende Dokumentation zum ordnungsgemäßen Ausführen der jeweiligen Verfahrensschritte kann dann in einem entsprechenden Speichermedium abgelegt werden. Das Dokumentationssystem ist über das Interface 1006 kommunikativ mit einem Eingabemittel 2000 (z.B. Tastatur, Maus, Touchscreen, etc.) gekoppelt, welches es dem Benutzer ermöglicht, dem Dokumentationssystem eine Freigabeentscheidung für entsprechend ordnungsgemäß ausgeführte Schritte des validierten Verfahrens mitzuteilen. Eine Freigabeentscheidung kann dabei in ihrer einfachsten Form als eine 1 Bit-Variable implementiert werden, wobei z.B. die "0" für "keine Freigabe" und die "1" für "Freigabe erteilt" steht. Beliebige andere, auch komplexere Freigabeformate sind möglich.

Erfindungsgemäß umfasst das Computersystem weiterhin ein Auswertungsmodul 1005, welches so konfiguriert, dass es eine Annahme der Freigabeinstruktion zumindest teilweise verhindert, wenn zumindest ein Messwert M1, M2 eines für das validierte Verfahren relevanten technischen Parameters außerhalb eines vordefinierten Toleranzbereichs liegt. Der vordefinierte Toleranzbereich kann z.B. Teil einer entsprechenden Arbeitsanweisung sein oder in beliebiger anderer Form, wie beispielsweise über entsprechende Tabellen oder dynamisch mittels einer Formel definiert sein. Nicht freigegebene Teilschritte des validierten Verfahren erfordern eine weitere Bearbeitung, ohne die eine vollständige Freigabe nicht erfolgen kann.

Wenn nun einer der empfangenen relevanten technischen Parameter außerhalb des vordefinierten Toleranzbereichs für den entsprechenden Messwert liegt, verweigert das Computersystem die Annahme einer positiven Freigabeentscheidung des Benutzers zumindest in Bezug auf den Schritt des validierten Verfahrens, bei dem die Abweichung des Messwerts auffällig wurde und daher möglicherweise dieser Schritt nicht ordnungsgemäß ausgeführt wurde. Das System verhindert damit eine unqualifizierte Freigabeentscheidung des Benutzers, die aufgrund einer falschen Beurteilung der technischen Messparameter durch den Benutzer zur Freigabe von eventuell nicht ordnungsgemäß aufbereiteten Medizinprodukten geführt hätte und damit das Risiko einer Patientenkontamination hervorrufen könnte. Beispielsweise kann die teilweise Verhinderung der Freigabe dem Benutzer durch eine entsprechende Warnmeldung angezeigt werden (z.B. "Die Reinigungsleistung zeigt eine unzureichende Wirkung an. Nachkontrolle mit Indikatoren erforderlich.")

Das Computersystem 1000 kann weiterhin ein Darstellungsmodul 1003 umfassen, welches konfiguriert ist, einen zeitlichen Messverlauf für mindestens einen der technischen Parameter in Echtzeit darzustellen. Das Darstellungsmodul 1003 ist mit dem Aufzeichnungsmodul 1001 kommunikativ gekoppelt 1007 und verarbeitet die aufgezeichneten Messdaten M1, M2 in einer Weise, dass sie grafisch, z.B. mittels der Ein-/Ausgabemittel 2000 (z.B. über Bildschirm, Plotter) an den Benutzer in Echtzeit ausgegeben werden können. Somit hat der Benutzer auch die Möglichkeit, den ordnungsgemäßen Verlauf des Aufbereitungsverfahrens visuell wahrzunehmen und zu überwachen. Bei groben Abweichungen der Messwerte vom zu erwartenden Messverlauf kann der Benutzer somit manuell eingreifen, und den entsprechenden Verfahrensschritt ggf. abbrechen und neu starten. Das validierte Verfahren wird auf diese Weise beschleunigt, da nicht erst mit der Annahmeverweigerung der Freigabeentscheidung eine Wiederholung des nicht ordnungsgemäß ausgeführten Schritts getriggert wird. Damit wird auch letztendlich der Energieverbrauch des gesamten validierten Verfahrens reduziert, da die Maschinenlaufzeit der Aufbereitungsmittel nicht für bereits fehlerhafte Aufbereitungsschritte vergeudet wird.

In einer weiteren Ausführungsform umfasst das Computersystem 1000 weiterhin ein Bibliotheksmodul 1004 und ein Auswertungsmodul 1005. Das Bibliotheksmodul 1004 hält für die verwendeten Aufbereitungsmittel 2001, 2002 wählbare Programme in einem Speicherbereich des Computersystems vor, wobei ein wählbares Programm einen zu erwartenden zeitlichen Messverlauf für mindestens einen der relevanten technischen Parameter enthält. Für jedes Aufbereitungsmittel kann dabei eine Vielzahl an Programmen abgelegt sein. Beispielsweise kann für einen Sterilisator für jede Risikoklasse von Medizinprodukten ein entsprechendes Programm hinterlegt sein. Letztendlich wäre es auch möglich, ein eigenes Programm pro Medizinprodukt zu hinterlegen. Für das Aufbereitungsverfahren wird das entsprechende Programm entweder vom Benutzer mittels der Ein-Ausgabemittel 2000 gewählt oder die Auswahl erfolgt automatisch, indem das entsprechende Aufbereitungsmittel (z.B. Sterilisator 2002) über seine Maschinenschnittstelle direkt oder indirekt mitteilt, welches Programm gerade erforderlich ist. Beispielsweise kann das Aufbereitungsmittel über eine Bilderkennungsfunktion oder anderweitige Erkennungsmechanismen wie RFID (radio frequencey identifier) oder Barcodes automatisch feststellen, welche Medizinprodukte gerade zur Aufbereitung anstehen und diese Information dem Bibliotheksmodul zur Auswahl des entsprechenden Programms übermitteln. Das Bibliotheksmodul kann dabei ggf. die Zuordnung des Medizinprodukts zu einer Risikoklasse ermitteln und dann das entsprechende Programm wählen. Die Zuordnung kann aus der zuvor beschriebenen Risikoklassenzuordnungsstruktur entnommen werden. Das Auswertungsmodul 1005 ist konfiguriert, einen Soll-Ist-Vergleich für mindestens einen zu erwartenden technischen Parameter eines gewählten Programms mit mindestens einem entsprechenden empfangenen Messwert auf der Basis des vordefinierten Toleranzbereichs durchzuführen. Somit kann das Auswertungsmodul in Echtzeit eine mögliche Abweichung der Messwerte vom zu erwartenden Messverlauf ermitteln, sobald auch nur ein einziger Messwert außerhalb des Toleranzbereichs definiert ist. Bei einer hohen Qualitätsanforderung an das validierte Verfahren ist der Toleranzbereich hinreichend klein zu wählen. Dies hat zur Konsequenz, dass bereits geringfügige Abweichungen der Messwerte als ein nicht ordnungsgemäßer Messverlauf zu werten sind. Solche geringfügigen Abweichungen wären für den Benutzer mittels der zuvor erläuterten visuellen Überwachungsmöglichkeit kaum erkennbar und bergen daher das Risiko, dass ein fehlerhafter Prozessschritt trotzdem bis zum Ende durchgeführt würde und erst am Ende eine Verweigerung der Freigabe durch das System erfolgen würde. Die Möglichkeit des maschinellen Soll-Ist-Vergleichs erlaubt, diese Aufbereitungsverzögerung zu verringern, da ein nicht ordnungsgemäßer Aufbereitungsschritt sofort durch das Auswertungsmodul erkannt wird und daraufhin ein Abbruch des gerade laufenden Aufbereitungsschritts automatisch durch das Computersystem erfolgen kann, indem beispielsweise eine entsprechende maschinenlesbare Instruktion über das Interface 1006 an das entsprechende Aufbereitungsmittel geschickt wird. Somit werden wiederum Zeit und Energie eingespart.

Der vordefinierte Toleranzbereich kann zudem in unterschiedliche Bereiche mit unterschiedlichen Risikoniveaus unterteilt sein. So kann beispielsweise ein Bereich "fehlerhaft" den Bereich Toleranzbereich beschreiben, bei dem definitiv ein nicht ordnungsgemäßer Ablauf des Aufbereitungsverfahrens vorliegt. Außerdem könnte es aber auch Bereiche für "kritisch" und "leicht kritisch" geben, in denen zwar das Aufbereitungsverfahren nicht optimal aber dennoch ausreichend läuft. Für diesen Fall sieht das Computersystem die Möglichkeit vor, eine Warnung zu generieren, wenn zumindest ein Messwert eines für das validierte Verfahren relevanten technischen Parameters in einen kritischen Bereich des vordefinierten Toleranzbereichs fällt. Die Warnung kann dabei von einem beliebigen Modul des Computersystems erzeugt werden, wie z.B. dem Auswertungsmodul 1005. Zur Übermittlung des Warnung an den Benutzer kann das Ein-/Ausgabemittel 2000 benutzt werden, um beispielsweise mittels eines Farbindikators oder eines Popup-Fensters dem Benutzer eine visuelle Warnung über einen Monitor zu übermitteln. Jegliche andere Art eines Warnsignals, z.B. in Form eines bestimmten Warntons (Klangbild) über einen Lautsprecher ist dabei ebenso möglich. Das Generieren einer Warnung für den Benutzer bei noch nicht fehlerhaften Abweichungen vom Optimalverlauf einer Messung erlaubt dem Benutzer ggf. in die Prozesssteuerung des betroffenen Aufbereitungsmittels korrigierend einzugreifen, was letztendlich einen drohenden Abbruch des Aufbereitungsverfahrens verhindert. Beispielsweise kann der Benutzer daraufhin manuell in die Steuerung der Betriebstemperatur oder des vorherrschenden Drucks im Aufbereitungsmittel eingreifen, um den gerade laufenden Aufbereitungsschritt wieder näher an zu erwartenden Messverlauf anzupassen. Dabei ist es auch möglich, die Warnung erst nach einem bestimmten Abweichverhalten bei den tatsächlich empfangenen Messwerten abzusetzen. Auf diese Weise würde ein einzelner Messwert, der außerhalb des entsprechenden Unterbereichs des vordefinierten Toleranzbereichs liegt, nicht gleich eine Warnung auslösen. Es könnte sich einfach um einen simplen Messfehler handeln. Wird jedoch ein Abweichungsmuster bei mehreren aufeinanderfolgenden Messwerten vom Auswertungsmodul detektiert, so erhöht sich die Wahrscheinlichkeit für ein tatsächliches Fehlverhalten des Aufbereitungsmittels. In diesem Fall sollte somit eine Warnung erzeugt werden. Der Fachmann kann verschiedenen Abweichungsmuster definieren, die unterschiedliche Warnstufen auslösen und dem Benutzer dabei unterstützen, das validierte Verfahren möglichst so zu kontrollieren, dass kein Abbruch erfolgt und der jeweilige Aufbereitungsschritt erfolgreich ordnungsgemäß abgeschlossen werden kann.

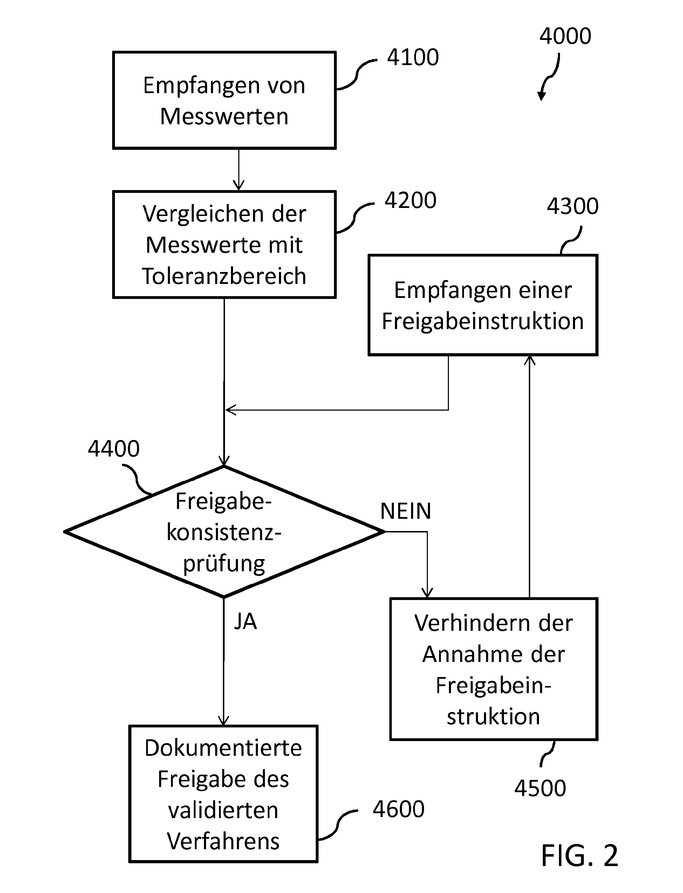

FIG. 2 ist ein Flussdiagramm zur Veranschaulichung eines erfindungsgemäßen Verfahrens 4000 zur Überwachung der Aufbereitung von Medizinprodukten nach einem validierten Verfahren, welches die folgenden Teile umfasst:

Empfangen 4100 von Messwerten von mindestens einem im validierten Verfahren verwendeten Aufbereitungsmittel, wobei die Messwerte für das validierte Verfahren relevante technische Parameter umfassen.

Vergleichen 4200 der empfangenen Messwerte mit einem entsprechenden vordefinierten Toleranzbereich.

Empfangen 4300 einer Freigabeinstruktion in Bezug auf mindestens eine Arbeitsanweisung hinsichtlich der verwendeten Aufbereitungsmittel des validierten Verfahrens.

Zumindest teilweises Verhindern 4500 einer Annahme der Freigabeinstruktion, wenn zumindest ein Messwert eines für das validierte Verfahren relevanten technischen Parameters außerhalb des vordefinierten Toleranzbereichs liegt.

Das Verhindern 4500 der Annahme der Freigabeinstruktion erfolgt für den Fall, dass eine Freigabekonsistenzprüfung 4400 nicht erfolgreich ist. Die Freigabekonsistenzprüfung 4400 umfasst die Überprüfung der Messwerte hinsichtlich ihrer Abweichungen vom zu erwartenden Messverlauf. Für den Fall, dass die Konsistenzprüfung erfolgreich ist (bei vorhandener Konsistenz), akzeptiert 4600 das Computersystem die Freigabeinstruktion des Benutzers und speichert die Dokumentation zur Freigabeentscheidung entsprechend im Dokumentationsmodul ab.

Das computerimplementierte Verfahren 4000 kann beispielsweise vom unter FIG. 1 beschriebenen Computersystem ausgeführt werden, wenn das Computersystem ein Computerprogramm mit Instruktionen abarbeitet, die den Verfahrensalgorithmus implementieren.

Das Vergleichen der Messwerte 4200 kann auch dazu dienen, den Erfolg eines Schrittes des validierten Verfahrens durch einen objektivierten Prüfindikator zu kontrollieren. In diesem Fall wird beim Durchführen eines Aufbereitungsschritts dem jeweiligen Aufbereitungsmittel ein sogenannter Prüfindikator verwendet. Der Zustand des Prüfindikators, häufig auch Prüfkörper genannt, stellt einen weiteren möglichen Messwert eines technischen Parameters des validierten Verfahrens dar. Dieser Messwert wird typischerweise nicht über eine Maschinenschnittstelle direkt vom Aufbereitungsmittel an das Aufzeichnungsmodul des Computersystem übermittelt sondern manuell vom Benutzer über eine Benutzerschnittstelle, wie z.B. die Ein-Ausgabemittel (cf. FIG. 1, 2000) erfasst. Die ISO-Norm 11140 legt z.B. Leistungsanforderungen und Prüfverfahren für chemische Indikatoren fest, die zur Verwendung bei Sterilisationsverfahren vorgesehen sind, bei denen Dampf, trockene Hitze, Ethylenoxid, γ- oder β -Strahlung, Dampf mit Formaldehydzusatz, gasförmiges Formaldehyd oder verdampftes Wasserstoffperoxid angewendet werden. Beispiele für Prüfindikatoren sind: Testkörper zur Kontrolle der Reinigungsleistung von Waschdesinfektionsautomaten; Edelstahlplättchen mit kombinierter Testanschmutzung in Schutzfolie; Edelstahlprüfkörper elektropoliert innen und außen, Luer-Lock-Anschluss; Edelstahlprüfkörper mit Silikonschlauch; etc. Ein Prüfindikator kann dabei bei einem entsprechenden Schritt des Aufbereitungsverfahrens (z.B. Ultraschall, maschinelle Reinigung, Sterilisation) mit in das verwendetet Aufbereitungsmittel gegeben werden und gibt dann zusätzliche Information über die erfolgreiche Durchführung des Schritts. Dies kann beispielsweise durch eine entsprechende Verfärbung des Prüfkörpers erfolgen. Der Prüfindikatorstatus nach dem Aufbereitungsschritt kann dann in manchen Fällen direkt vom Benutzer erkannt werden und dem Computersystem als ein relevanter technischer Parameter des validierten Verfahrens mitgeteilt werden. In anderen Fällen wird eine Analyse des Prüfindikatorstatus von einer anderen Stelle durchgeführt, ohne dass der Benutzer direkt den Prüfindikatorstatus erfährt. Beispielsweise kann der Prüfindikatorstatus dem Benutzer in Form eines Codes übermittelt werden. Der Code kann dabei z.B. eine einmalige, individuelle, speziell kodierte Folge von ASCII-zeichen sein. Jede andere geeignete Kodierung ist ebenfalls möglich. Der Benutzer kann dann dem Computersystem den übermittelten Code (kodierter Prüfindikatorstatus) durch eine entsprechende Eingabe mitteilen. Das Computersystem hat die entsprechende Information, die zum Dekodieren des Codes verwendet werden kann, gespeichert und kann den Code damit entschlüsseln, um letztendlich den Prüfindikatorstatus zu erhalten. Dieser kann nun gegebenenfalls dem Benutzer angezeigt werden. Allerdings kann der Benutzer den Prüfindikatorstatus nicht manipulieren. Damit wird sichergestellt, dass ein fehlgeschlagener Schritt im validierten Verfahren, der mittels Prüfindikatorstatus nachgewiesen werden konnte, immer zu einer Verhinderung einer vollständigen Freigabe führen wird, da der Benutzer diese Blockadewirkung des Computersystems nicht umgehen kann.

Um eine passende Vergleichsgrundlage für einen Soll-Ist-Vergleich für mindestens einen zu erwartenden technischen Parameter zu ermöglichen, kann entweder auf Veranlassung durch den Benutzer oder automatisch auf Veranlassung durch das verwendete Aufbereitungsmittel, ein entsprechendes Programm ausgewählt werden. Das ausgewählte Programm enthält einen zu erwartenden zeitlichen Messverlauf für mindestens einen der relevanten technischen Parameter. Der Soll-Ist-Vergleich kann dann durch Vergleich der tatsächlich empfangenen Messwerte mit den zu erwartenden Werten in Echtzeit durchgeführt werden. Dabei wird auch überprüft, ob mindestens einer der empfangenen Messwerte eventuell außerhalb des vordefinierten Toleranzbereichs liegt. Ist dies der Fall, kommt das Auswertungsmodul ggf. zum Ergebnis, dass die Freigabekonsistenzprüfung nicht erfolgreich war. Wie weiter oben beschrieben, kann als Grundlage für die Freigabekonsistenzprüfung 4400 aber auch ein Mehrzahl an aufeinanderfolgenden Messwerten dienen, so dass eine erfolglose Freigabekonsistenzprüfung (bei fehlender Konsistenz) ein bestimmtes Abweichungsverhalten mehrerer Messwerte von den zu erwartenden Werten über einen gewissen Zeitraum erfordert. Für den Fall, dass der Toleranzbereich wie unter FIG. 1 erläutert in mehrere Teilbereiche untergliedert ist, kann auf Basis des Messwertvergleichs eine Warnung generiert werden, wenn zumindest einer der Messwerte eines für das validierte Verfahren relevanten technischen Parameters in einen kritischen Teilbereich des vordefinierten Toleranzbereichs fällt.

Beim Einsatz von Prüfindikatoren sind in der Regel bestimmte Zeitintervalle vorgeschrieben, innerhalb derer eine Prüfung mittels Prüfindikator durchgeführt werden muss. Ein solches Zeitintervall kann ebenfalls als vordefinierter Toleranzbereich im Sinne der Erfindung gesehen werden. Ist beispielsweise der Einsatz eines Prüfindikators mindestens alle 180 Tage erforderlich, so kann 150 Tage nach dem letzten Einsatz eine Warnung erster Stufe generiert werden, z.B. gelbe Anzeige der noch verbleibenden Tage bis zum nächsten Einsatz. Nach 170 Tagen kann die zweite Warnstufe erreicht werden und die Farbe wechselt auf Rot. Nach 180 Tagen könnte rot blinkend angezeigt werden, dass das entsprechende Aufbereitungsmittel nicht mehr verwendbar ist, bis erneut ein Lauf mit Prüfindikator durchgeführt wurde. Der Toleranzbereich wäre hiermit überschritten und eine eventuelle Freigabeinstruktion vom Benutzer wird vom Auswertungsmodul nicht angenommen.

FIG. 3. zeigt eine Darstellung 100 von aufgezeichneten von einem Aufbereitungsmittel empfangenen Messwerten relevanter technischer Verfahrensparameter 101, 102, 103. Die Grafik zeigt als Beispiel Messwerte eines Sterilisators. Die Messdaten der Messkurve 101 entsprechen dem Druckverlauf im Sterilisator über die Zeit 103. Die Messdaten der Messkurve 102 entsprechen dem Temperaturverlauf über die Zeit 103. Das Beispiel zeigt einen ordnungsgemäßen Ablauf des Sterilisationsschritts als Teil des validierten Verfahrens. Diese Daten könnten ebenso als die zu erwartenden Messwerte für das Aufbereitungsmittel Sterilisator für eine bestimmte Risikoklasse von Medizinprodukten in einem entsprechenden Programm des Bibliothekmoduls hinterlegt sein und als Vergleichsdaten für den Soll-Ist-Vergleich der Auswertungsmoduls verwendet werden. Dabei könnte zu jedem Zeitpunkt ein Toleranzbereich mit dem jeweiligen zu erwartenden Wert assoziiert sein. im Beispiel stellt T2 einen in einem bestimmten Zeitpunkt vordefinierten Toleranzbereich für den Temperaturverlauf während der Abkühlphase dar und T1 einen vordefinierten Toleranzbereich für den Druckverlauf. Die Toleranzbereiche können für jeden technischen Parameter über den gesamten Messverlauf hin variieren. Beispielsweise kann bei hohen Temperaturen der Toleranzbereich zu höheren Temperaturen hin größer sein, da eine höhere Temperatur nicht schädlich für den Erfolg ist. Jedoch kann der Toleranzbereich nach unten enger limitiert sein, da immer eine gewisse Mindesttemperatur erforderlich ist. Auch in den verschiedenen Aufheiz- bzw. Abkühlzyklen können unterschiedliche Toleranzbereiche existieren.

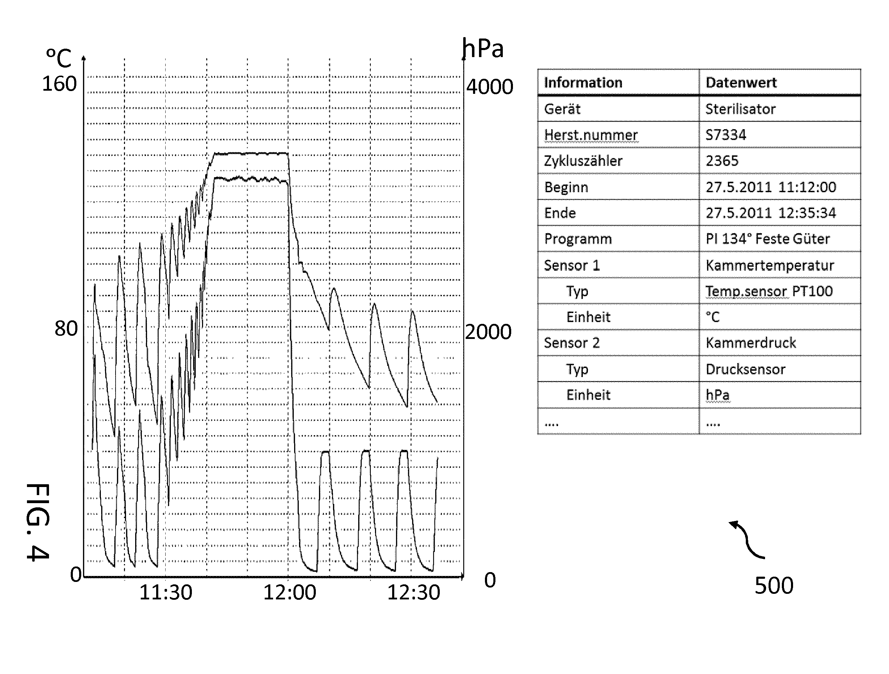

FIG. 4 ist eine schematische Darstellung eines beispielhaften grafischen Benutzerinterfaces 500 des erfindungsgemäßen Computersystems. Das Darstellungsmodul kann solche grafischen Benutzerinterfaces erzeugen und über die Ein-/Ausgabemittel ggf. in Echtzeit zur Anzeige für den Benutzer bringen. Im linken Teil des Interfaces findet der Benutzer die Darstellung der aufgezeichneten Messwerte. Der rechte Teil beinhaltet in Tabellenform Metadaten, die die Messergebnisse eindeutig klassifizieren. Mit einer Freigabeentscheidung des Benutzers werden letztendlich alle in den Aufbereitungsprozess eingebundenen Schritte und Aufbereitungsmittel (z.B. Reinigungsvorrichtung, Siegelgerät und Sterilisator) mit dem jeweiligen Messablauf der relevanten technischen Parameter als Echtzeitgrafik und den entsprechenden Pflichtangaben zusammengeführt, dokumentiert und archiviert. Die Freigabeentscheidung kann die Eingabe einer erweiterten digitalen Signatur des Benutzers erfordern, die den Benutzer zur Freigabe autorisiert. Die Zusammenführung der Informationen kann in einem Dokument in einem nichtänderbaren Dateiformat, wie z.B. einem pdf-Dokument, vollzogen werden. Aus diesen Informationen können dann beispielsweise Indikatoren abgeleitet werden, die die aufbereiteten Medizinprodukte kennzeichnen und eine genau Rückverfolgung eines jeden Medizinprodukts hinsichtlich seiner Aufbereitungshistorie ermöglichen. Solche Indikatoren können z.B. als Barcodeetiketten gedruckt werden, wobei der Barcode ausreichend Information kodiert, um eine spätere Zuordnung in den archivierten Daten zu ermöglichen. Eine alternative Variante stellt das Erstellen von RFID Tags dar, die mit den entsprechenden Metainformationen kodiert sind und den aufbereiteten Medizinprodukten angeheftet (z.B. aufgeklebt) werden.

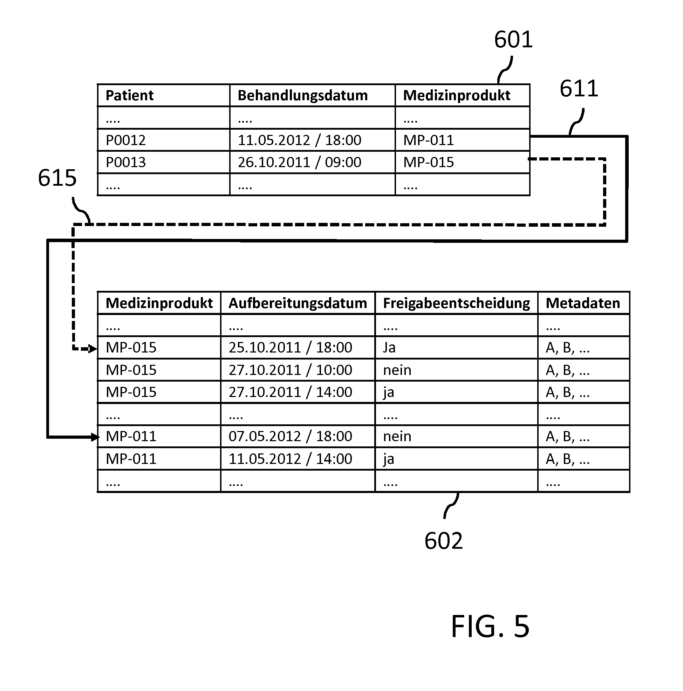

FIG. 5 zeigt vereinfachte Datenstrukturen 601, 602, die im erfindungsgemäßen Computersystem gekoppelt 611, 615 sind. Die erste Datenstruktur 601 wird verwendet, um die Anwendung eines Medizinprodukts am Patienten zu protokollieren. Die Abbildung zeigt beispielhaft drei mögliche Felder (Patient, Behandlungsdatum, Medizinprodukt), die als Datensatz bei jeder Behandlung eines Patienten im Computersystem gespeichert werden können. Im Beispiel wurde Patient P0012 am 11. Mai 2012 um 14:00 Uhr mit dem Medizinprodukt MP-011 behandelt. Patient P0013 wurde am 26. Oktober 2011 um 09:00 Uhr mit dem Medizinprodukt MP-015 behandelt. Diese erste Datenstruktur 601 ist nun mit einer weiteren Datenstruktur 602 mit Daten des Aufzeichnungsmoduls und des Dokumentationsmoduls in Bezug auf die Aufbereitung von Medizinprodukten gekoppelt 611, 615. Die Kopplung ist dabei derart gestaltet, dass ein Datensatz der Patientenbehandlungsdatenstruktur 601, bei dem ein bestimmtes Medizinprodukt (z.B. MP-011) verwendet wurde, entsprechenden Datensätzen für dieses Medizinprodukt in der Medizinproduktaufbereitungsdatenstruktur 602 zugeordnet werden kann. Dies kann beispielsweise über entsprechende Schlüsselfelder oder Zeigerstrukturen realisiert werden. Die weitere Datenstruktur 611 (Medizinproduktaufbereitungsdatenstruktur) beinhaltet für die beiden Medizinprodukte MP-011 und MP-015, die bei den Patientenbehandlungen von P0012 und P0013 zum Einsatz kamen, Informationen in Bezug auf die entsprechenden Aufbereitungsschritte. Dabei wird für das Medizinprodukt ausgewiesen, wann eine Aufbereitung erfolgte (Aufbereitungsdatum) und ob diese erfolgreich war (Freigabeentscheidung). Außerdem sind eine Reihe von Metadaten A,B, ..., die Details (z.B. relevante technische Parameter) über den jeweiligen Aufbereitungsprozess enthalten, mit abgespeichert. Anhand der Kopplung der beiden Datenstrukturen 601, 602 kann nun für jede Anwendung des Medizinprodukts am Patienten die Konsistenz der Freigabeentscheidung und den entsprechenden für das validierte Verfahren relevanten technischen Parametern überprüfbar werden. So lässt sich im Beispiel nachweisen, dass das Medizinprodukt MP-015 ordnungsgemäß am 25.10.2011 um 18:00 freigegeben wurde. Es war also bei seinem Einsatz am 26.10.2011 bei der Behandlung von P0013 in einem einwandfreien Zustand. Allerdings schlug die darauf folgende Aufbereitung am 27.10.2011 zunächst fehl, was aber noch am selben Tag korrigiert wurde. Die Aufbereitung des Medizinprodukts MP-011 wurde am 7.5.2012 zunächst nicht ordnungsgemäß freigegeben. Bei einer erneuten Versuch am 11.5.2012 um 14:00 Uhr konnte dann die Aufbereitung ordnungsgemäß abgeschlossen werden, was zu einer positiven Freigabeentscheidung führte. MP-011 fand dann ordnungsgemäß am 11.5.2012 um 18:00 Uhr Verwendung bei der Behandlung von P0013. Diese lückenlose Dokumentation in Verbindung mit dem Verhindern einer vollständigen Freigabe bei nicht erfolgreicher Aufbereitung erlaubt also zu jedem Zeitpunkt sicherzustellen, dass nur ordnungsgemäß freigegebene Medizinprodukte zum Einsatz kommen und dies auch jederzeit nachweisbar ist.

Mittels der beiden gekoppelten bzw. verlinkten Datenstrukturen 601, 602 kann also jederzeit lückenlos nachgewiesen werden, dass sich bei einer Patientenbehandlung die verwendeten Medizinprodukte in einem einwandfreien Zustand befanden. Dies kann in einem entsprechenden graphischen Benutzerinterface mittels bekannter Drill-Down Techniken implementiert werden. Der Benutzer kann dabei z.B. über eine Sicht auf die Behandlungstermine einzelner Patienten durch einfache Navigationsmechanismen, wie z.B. Doppelklick oder "mouse over" auf einen Patienten oder Behandlungstermin, die Anzeige der entsprechenden Daten für die jeweiligen Medizinprodukte bewirken.

Ausführungsformen der Erfindung können in Form von digitalen Schaltkreisen, Computerhardware, Firmware, Software oder in beliebigen Kombinationen davon implementiert werden. Die Erfindung kann weiterhin in Form eines Computerprogrammprodukts, z.B. eines Computerprogramms auf einem physischen Informationsträger (z.B., maschinenlesbares Speichermedium) implementiert werden, um von einer Datenverarbeitungsvorrichtung (z.B., programmierbarer Prozessor, Computer oder kommunikativ gekoppelte Computer) ausgeführt zu werden oder deren Betrieb zu steuern. Ein Computerprogrammprodukt wie beansprucht kann in einer beliebigen Programmiersprache erstellt sein, wobei auch kompilierte oder interpretierte Sprachen eingeschlossen sind. Es kann in beliebiger Form eingesetzt werden, beispielsweise als alleinstehendes Programm, Modul, Komponente, Unterprogramm oder als andere Einheit, die geeignet ist, um in einem Datenverarbeitungssystem benutzt zu werden. Das Computerprogramm kann von einem Computer ausgeführt werden oder aber auch durch mehrere über ein Kommunikationsnetzwerk miteinander verbundene Computer entweder an einem Ort oder über mehrere Orte verteilt. Ein computerimplementiertes Verfahren kann durch das Ausführen entsprechender Computerprogrammprodukte auf entsprechenden Datenverarbeitungsvorrichtungen ausgeführt werden.

Verfahrensschritte gemäß der Erfindung können von einem oder mehreren programmierbaren Prozessoren durch abarbeiten des Computerprogramms ausgeführt werden, um die erfindungsgemäßen Funktionen auszuführen, wobei Eingangsdaten verarbeitet werden und entsprechende Ausgangsdaten dabei erzeugt werden. Die Verfahrensschritte können auch durch spezielle Logikbausteine wie z.B. field programmable gate arrays (FPGA) oder application-specific integrated circuits (ASIC) ausgeführt werden.

Beispiele für Prozessoren, die geeignet zur Ausführung des Computerprogramms sind, beinhalten allgemeine oder spezialisierte Mikroprozessoren und jegliche Ein- oder Mehrprozessorlösung eines beliebigen digitalen Computers. Im allgemeinen erhält eine Prozessor Instruktionen und Daten von einem Read-Only Memory (ROM) oder Random Access Memory (RAM) oder von beiden. Die wesentlichen Elemente eines Computers sind mindestens ein Prozessor und ein oder mehrere Speichermedien, um Daten und Instruktionen zu speichern. Im Allgemeinen ist ein Computer auch mit einem oder mehreren Massenspeichermedien (z.B. magnetische, magneto-optische, optische oder solid state (SSD) Speichermedien) gekoppelt, um davon Daten zu empfangen oder Daten dort abzuspeichern. Solche Speichermedien können auch bei Bedarf (on demand) bereitgestellt werden oder über das Internet erreichbar sein (z.B. Cloud Computing). Geeignete Datenträger zum Abspeichern von Programminstruktionen und Daten umfassen alle Arten von nicht-flüchtigen Speicherelementen wie Halbleiterspeicherelemente (z.B., EPROM, EEPROM), Flash-Memory Vorrichtungen, magnetische oder magneto-optische Speichermedien, CD-ROM, DVD-ROM oder Blue-Ray Disks. Die Prozessor- und Speicherelemente können ergänzt werden durch spezielle Logikbausteine, oder auch Teil dieser sein.

Um die Interaktion mit dem Benutzer zu ermöglichen, kann die Erfindung auf einem Computer implementiert sein, der mindestens eine Ausgabevorrichtung (z.B., LCD Monitor, Lautsprecher, etc.) und mindestens eine Eingabevorrichtung (z.B. Tastatur, Touchscreen, Mikrofon, Zeigevorrichtung wie Maus oder Trackball) umfasst.

Der Erfindung kann auf einer Datenverarbeitungsvorrichtung implementiert werden, die eine Backend-Komponente (z.B. Datenserver) umfasst, oder eine Middleware-Komponente (z.B. Anwendungsserver), oder eine Frontend-Komponente (z.B. Client-Computer mit graphischem Benutzerinterface oder Webbrowser) worüber der Benutzer mit einer Ausführungsform der Erfindung interagieren kann, oder jegliche Kombination von Backend-, Middleware- und Frontendkomponenten.

Clientcomputer können auch mobile Endgeräte sein wie beispielsweise Smartphones, Tablet PCs oder jede beliebige tragbare Computervorrichtung. Die Komponenten des Systems können miteinander kommunikativ gekoppelt sein (z.B. mittels eines Kommunikationsnetz Werks wie Local Area Network LAN oder Wide Area Network WAN, Internet oder drahtlosen LAN oder Telekommunikationsnetzwerken).

Das Computersystem kann Clients und Server umfassen. Ein Client und ein Server sind im Allgemeinen physisch voneinander entfernt und interagieren über ein Kommunikationsnetzwerk. Die Beziehung zwischen Client und Server entsteht dabei durch Computerprogramme, die auf den jeweiligen Computern ausgeführt werden und die eine Client-Server Beziehung untereinander haben.

## Patentansprüche

1. Computersystem (1000) zur Überwachung der Aufbereitung von Medizinprodukten nach einem validierten Verfahren, umfassend:
ein Aufzeichnungsmodul (1001), welches konfiguriert ist Messwerte (M1, M2) von mindestens einem im validierten Verfahren verwendeten Aufbereitungsmittel (2001, 2002) zu empfangen, wobei die Messwerte für das validierte Verfahren relevante technische Parameter (101, 102) umfassen;
ein Dokumentationsmodul (1002) zum Erfassen mindestens einer Freigabeinstruktion für eine Freigabeentscheidung in Bezug auf mindestens eine Arbeitsanweisung hinsichtlich der verwendeten Aufbereitungsmittel (2001, 2002) des validierten Verfahrens;
**gekennzeichnet dadurch, dass**
ein Auswertungsmodul (1005) konfiguriert ist, die Annahme der Freigabeinstruktion zumindest teilweise zu verhindern, wenn zumindest ein Messwert (M1, M2) eines für das validierte Verfahren relevanten technischen Parameters (101, 102) außerhalb eines vordefinierten Toleranzbereichs liegt.

2. Computersystem nach Anspruch 1, wobei die Messwerte (M1, M2) mindestens einen der folgenden für das validierte Verfahren relevanten technischen Parameter umfassen: Druck (101), Temperatur (102), Zeitdauer (103), Prüfindikatorzustand.

3. Computersystem nach einem der vorhergehenden Ansprüche, weiterhin umfassend:
ein Darstellungsmodul, welches konfiguriert ist, einen zeitlichen Messverlauf (100) für mindestens einen der technischen Parameter (101, 102) in Echtzeit darzustellen.

4. Computersystem nach einem der vorhergehenden Ansprüche, weiterhin umfassend:
ein Bibliotheksmodul (1004), welches für die verwendeten Aufbereitungsmittel (2001, 2002) wählbare Programme vorhält, wobei ein wählbares Programm einen zu erwartenden zeitlichen Messverlauf für mindestens einen der relevanten technischen Parameter (101, 102) enthält; und
das Auswertungsmodul (1005) konfiguriert ist, einen Soll-Ist-Vergleich für mindestens einen zu erwartenden technischen Parameter eines gewählten Programms mit mindestens einem entsprechenden empfangenen Messwert auf der Basis des vordefinierten Toleranzbereichs durchzuführen.

5. Computersystem nach einem der vorhergehenden Ansprüche, wobei das Aufzeichnungsmodul (1001) die Messwerte (M1, M2) über eine Maschinenschnittstelle vom verwendeten Aufbereitungsmittel (2001, 2002) empfängt.

6. Computersystem nach einem der Ansprüche 1 bis 4, wobei das Aufzeichnungsmodul (1001) die Messwerte über eine Benutzerschnittstelle von einem Benutzer empfängt.

7. Computersystem nach einem der vorhergehenden Ansprüche, wobei ein Modul konfiguriert ist, eine Warnung zu generieren, wenn zumindest ein Messwert (M1, M2) eines für das validierte Verfahren relevanten technischen Parameters in einen kritischen Bereich des vordefinierten Toleranzbereichs fällt.

8. Computersystem nach Anspruch 7, wobei die generierte Warnung in einem oder mehreren der folgenden Formate angezeigt wird: Farbindikator, Klangbild, und Popup-Fenster.

9. Computersystem nach einem der vorhergehenden Ansprüche, wobei weiterhin eine Datenstruktur (601) verwendet wird, um die Anwendung eines Medizinprodukts am Patienten zu protokollieren und wobei diese Datenstruktur (601) mit einer weiteren Datenstruktur (602) mit Daten des Aufzeichnungsmoduls und des Dokumentationsmoduls gekoppelt (611, 615) ist, so dass für jede Anwendung des Medizinprodukts am Patienten die Konsistenz der Freigabeentscheidung und den entsprechenden für das validierte Verfahren relevanten technischen Parametern überprüfbar ist.

10. Computerimplementiertes Verfahren (4000) zur Überwachung der Aufbereitung von Medizinprodukten nach einem validierten Verfahren, umfassend:
Empfangen (4100) von Messwerten (M1, M2) von mindestens einem im validierten Verfahren verwendeten Aufbereitungsmittel (2001, 2002), wobei die Messwerte für das validierte Verfahren relevante technische Parameter (101, 102) umfassen;
Vergleichen (4200) der empfangenen Messwerte mit einem entsprechenden vordefinierten Toleranzbereich;
Empfangen (4300) einer Freigabeinstruktion für eine Freigabeentscheidung in Bezug auf mindestens eine Arbeitsanweisung hinsichtlich der verwendeten Aufbereitungsmittel (2001, 2002) des validierten Verfahrens; und
zumindest teilweises Verhindern (4500) der Annahme der Freigabeinstruktion, wenn zumindest ein Messwert (M1, M2) eines für das validierte Verfahren relevanten technischen Parameters (101, 102) außerhalb des vordefinierten Toleranzbereichs liegt.

11. Computerimplementiertes Verfahren (1000) nach Anspruch 11 weiterhin umfassend:
Kontrollieren des Erfolgs eines Schrittes des validierten Verfahrens durch einen objektivierten Prüfindikator.

12. Computerimplementiertes Verfahren (1000) nach Anspruch 10 oder 11, weiterhin umfassend:
Auswählen eines Programms für ein verwendetes Aufbereitungsmittel (2001, 2002), wobei das ausgewählte Programm einen zu erwartenden zeitlichen Messverlauf für mindestens einen der relevanten technischen Parameter (101, 102) enthält; und
Durchführen eines Soll-Ist-Vergleichs für mindestens einen zu erwartenden technischen Parameter des gewählten Programms mit mindestens einem entsprechenden empfangenen Messwert auf der Basis des vordefinierten Toleranzbereichs.

13. Computerimplementiertes Verfahren (1000) nach Anspruch 12, wobei der Soll-Ist-Vergleich in Echtzeit durchgeführt wird.

14. Computerimplementiertes Verfahren (1000) nach einem der Ansprüche 10 bis 13 weiterhin umfassend:
Generieren einer Warnung, wenn zumindest ein Messwert (M1, M2) eines für das validierte Verfahren relevanten technischen Parameters in einen kritischen Bereich des vordefinierten Toleranzbereichs fällt.

15. Computerprogrammprodukt umfassend Instruktionen, welche beim Laden in mindestens ein Speichermodul und Abarbeiten durch mindestens einen Prozessor das computerimplementierte Verfahren nach einem der Ansprüche 10 bis 14 ausführen.
